# EUROPEAN PATENT SPECIFICATION

(11) **EP 1 634 964 B1**
(45) Date of publication and mention of the grant of the patent: **25.06.2008**
(21) Application number: 05016591.9
(22) Date of filing: 29.07.2005
(51) Int. Cl.: C12Q 1/68

(54) **Method for determining protein binding sites in genomic DNA**
Verfahren zur Bestimmung von Protein-bindenden Stellen in genomischer DNS
Procédé pour la détermination de sites qui se lient aux protéines dans l'ADN génomique

(30) Priority: 09.09.2004 JP 2004262045
(43) Date of publication of application: 15.03.2006
(73) Proprietor: HITACHI SOFTWARE ENGINEERING CO., LTD., Yokohama-shi, Kanagawa 230-0045 (JP)
(72) Inventor: Fujisaki, Ayako, Shinagawa-ku, Tokyo 140-0002 (JP)
(74) Representative: Liesegang, Roland

(56) References cited:
- WO-A-01/85915
- WO-A-02/059371
- REN B ET AL: "Genome-wide location and function of DNA binding proteins" CELL, CELL PRESS, CAMBRIDGE, NA, US, vol. 290, December 2000 (2000-12), pages 2306-2309, XP002170840 ISSN: 0092-8674
- DATABASE WPI Section Ch, Week 200404 Derwent Publications Ltd., London, GB; Class B04, AN 2004-038389 XP002399694 -& JP 2003 279568 A (HITACHI SOFTWARE ENG CO LTD) 2 October 2003 (2003-10-02)
- LIU X SHIRLEY ET AL: "An algorithm for finding protein-DNA binding sites with applications to chromatin-immunoprecipitation microarray experiments" NATURE BIOTECHNOLOGY, vol. 20, no. 8, August 2002 (2002-08), pages 835-839, XP002399182 ISSN: 1087-0156
- MACISAAC KENZIE D ET AL: "A hypothesis-based approach for identifying the binding specificity of regulatory proteins from chromatin immunoprecipitation data" BIOINFORMATICS (OXFORD), vol. 22, no. 4, February 2006 (2006-02), pages 423-429, XP002399183 ISSN: 1367-4803

## Description

### BACKGROUND OF THE INVENTION

### Field of the Invention

The present invention relates to a method for identifying protein binding sites on genomic DNA, using a DNA chip.

### Background Art

Gene expression is regulated by a sequence-specific binding of a plurality of proteins to genomic DNA. Examining gene expression regulation is equivalent to exploring the mechanism inherent in living organisms of development, differentiation, growth, and aging. For example, it is known that the binding of a transcription factor called "activator" (protein) and a genomic sequence performs an important function for the maintenance of life. Generally, transcription factors are said to bind to a promoter region located upstream of a gene to activate the expression of a gene downstream. However, transcription factors do not always bind to an upstream region of a gene, and it has been reported that they also bind to regions called enhancers or silencers several hundred or a thousand bases away and perform regulation.

Thanks to the development of DNA chips, methods have been established in recent years that allow gene expression of various life forms to be examined in a comprehensive manner. In a hybridization technique, the cDNA sequence or oligonucleotides as part of a gene (coding region) are prepared and spotted on a slide glass as a probe in a dense manner. mRNA is collected from a specific living species and added to the probe as a target, whereby hybridization occurs. Such hybridization techniques now enable the measurement of transcript levels in a comprehensive manner.

Patent Document 1 discloses a method for analyzing the interaction of protein and DNA using the aforementioned technique. In this method, DNA reproducing a noncoding region (intergenic region) is spotted on a slide glass, thereby preparing a DNA chip having the noncoding region as a probe. Meanwhile, protein and DNA are cross-linked *in vivo* using formaldehyde, and after the cells have been destroyed, chromatin immunoprecipitation is conducted using an antibody that recognizes a specific transcription factor (protein). As a result, a DNA fragment including a noncoding region to which the specific transcription factor can bind can be obtained as a target. The DNA fragment is labeled by fluorescent dye and caused to hybridize to a DNA chip in which the noncoding region is disposed as a probe. In this way, control genes in the transcription factor can be examined comprehensively.

Patent Document 2 discloses a method for identifying a protein-binding region using a DNA chip in which a coding region (gene) is spotted as a probe. In this method, DNA including a noncoding region and some of the coding regions on either side of the noncoding region is used as a target. The method enables the determination of the presence of a protein-binding region between the two adjacent coding regions. The method enables the detection of a DNA-protein binding region with little experimental error without requiring the renewed preparation of a DNA chip in which a noncoding region is used as a probe.

Other various methods for identifying protein-binding regions on DNA are also being studied using computers. Algorithms have also been developed that predict binding regions by sequential analysis based on statistical approaches.

Patent Document 3 discloses a program for identifying a protein-binding region based on a database in which information about known transcription factors are stored and a sequence database, using an algorithm with high computational efficiency. Binding sequences of transcription factors are short and recognized to be from 6 to 15 bases long. When such short sequences are detected from the entire noncoding regions of a genome, numerous false positives are found. Therefore, it is important in the prediction of protein-binding regions how to reduce the false positives. The publication discloses that statistically significant binding sequences are predicted using known information in a transcription factor database called TRANSFAC.
Patent Document 1: JP Patent Publication (Kohyo) No. 2003-508066 A
Patent Document 2: JP Patent Publication (Kokai) No. 2003-279568 A
Patent Document 3: JP Patent Publication (Kohyo) No. 2003-535394 A

### SUMMARY OF THE INVENTION

In the example of Patent Document 3, statistical prediction is made using only computers. Although the method reflects existing data, it cannot confirm the actual protein-binding regions in a comprehensive manner.

Promoter regions where RNA polymerases bind, or gene expression regions where transcriptional control factors (proteins) bind are referred to as cis-elements. In Patent Publications 1 and 2, cis-elements cannot be accurately identified; only binding regions are estimated.

Application of DNA chip technology allows for the narrowing of gene regulating regions in a comprehensive manner. However, no system has so far been devised that is equipped to find cis-elements from experimental data and analyze them accurately.

It is therefore an object of the invention to solve the aforementioned problems and provide a method for detecting DNA-protein binding regions using a DNA chip in which a noncoding or coding region is spotted.

The invention which is defined in the appended claims, provides a method for determining a protein binding site on a genomic DNA, comprising:
preparing a DNA fragment as a probe to be spotted on a DNA chip comprising a gene-noncoding region on said genomic DNA, preparing a fluorescently labelled DNA fragment as a target comprising a gene-noncoding region on genomic DNA, to which DNA fragment a specific protein is bound by cross-linking, extracting said DNA-fragment using an antibody specifically recognizing said specific protein, removing said cross-linking, separating said specific protein from said DNA fragment, labelling said DNA fragment with a fluorescent dye, thereby obtaining a target and causing said target to be hybridized to said probe, thereby obtaining first fluorescent intensity data;
preparing a DNA fragment as a probe to be spotted on said DNA chip comprising a gene coding region on said genomic DNA, preparing a fluorescently labelled DNA fragment as a target comprising a gene-noncoding region and part of coding regions on either side thereof on DNA, to which DNA fragment a specific protein is bound by cross-linking, extracting said DNA-fragment using an antibody specifically recognizing said specific protein, removing said cross-linking, separating said specific protein from said DNA fragment, labelling said DNA fragment with a fluorescent dye, thereby obtaining a target and then causing said target to be hybridized to said probe, thereby obtaining second fluorescent intensity data;
conducting a control test without using an antibody specifically recognizing said specific protein, by preparing a DNA fragment as a probe to be spotted on said DNA chip comprising a gene coding region on said genomic DNA, preparing a fluorescently labelled DNA fragment as a target comprising a coding region, causing said target to be hybridized to said probe, thereby obtaining fluorescent intensity data from said control test;
entering said first and said second fluorescent intensity data and information about said probes and said targets;
detecting a binding site on said genomic DNA of said probes to which said specific protein binds, based on said first and said second fluorescent intensity data and said information about said probes and said targets, and then visually displaying said binding site on the genome sequence of said probes;
detecting a candidate for a cis element on said genomic DNA to which said specific protein binds; and
detecting the frequency of appearance of said cis element in a gene-noncoding region on said genomic DNA.

In accordance with the invention, the position on a genomic DNA to which a specific protein binds, namely, a noncoding region, can be identified. Detecting gene controlled by a certain transcription factor (protein) in a comprehensive manner can lead not only to the clarification of changes in the expression of known control genes but also to the discovery of unknown gene regulation mechanisms. Further, by detecting specific DNA sequences recognized by specific proteins, the possibility of sequence-specific binding increases, so that light can be shed on transcription regulation of a variety of unknown genes.

### BRIEF DESCRIPTION OF THE DRAWINGS

Fig. 1 shows an example of a method for measuring hybridization intensities using a DNA chip.
Fig. 2 shows the outline of a protein binding site identifying system according to the invention.
Fig. 3 shows an example of the types of data in a chip database and the structure of data.
Fig. 4 shows an example of a screen displayed on a display unit after the entry of data.
Fig. 5 shows the overall flow of processes performed by a program in accordance with the invention.
Fig. 6 shows a dialog for entering settings for running a protein binding site display program.
Fig. 7 shows a first example of the results of running the protein binding site display program.
Fig. 8 shows a second example of the results of running the protein binding site displaying program.
Fig. 9 shows an example of the displayed results of running a program for displaying a protein binding site list.
Fig. 10 shows an example of the flow of processes performed by the protein binding site displaying program.
Fig. 11 shows another example of the flow of processes performed by the protein binding site displaying program.
Fig. 12 shows the flow of processes performed by the protein binding site list displaying program.
Fig. 13 shows a dialog for entering settings for running a cis-element search program.
Fig. 14 shows an example of the displayed results of running the cis-element search program.
Fig. 15 shows an example of the details of the results of running the cis-element search program.
Fig. 16 shows the flow of processes performed by the cis-element search program.
Fig. 17 shows a dialog for entering settings for running a program for detecting and displaying cis element appearance frequencies.
Fig. 18 shows an example of the displayed results of running the program for detecting and displaying cis element appearance frequencies.
Fig. 19 shows the flow of processes performed by the program for detecting and displaying cis element appearance frequencies.
Fig. 20 shows the flow of processes performed by a program for cis- element false-positive determination.

### DESCRIPTION OF THE PREFERRED EMBODIMENTS

Initially, a method for determining hybridization intensity, namely, fluorescent intensity, by a hybridization experiment using a DNA chip or DNA microarray is described. Hereafter, a DNA fragment affixed to the DNA chip (spotted on a slide glass) will be referred to as a probe, and a DNA fragment to be hybridized to the probe will be referred to as a target.

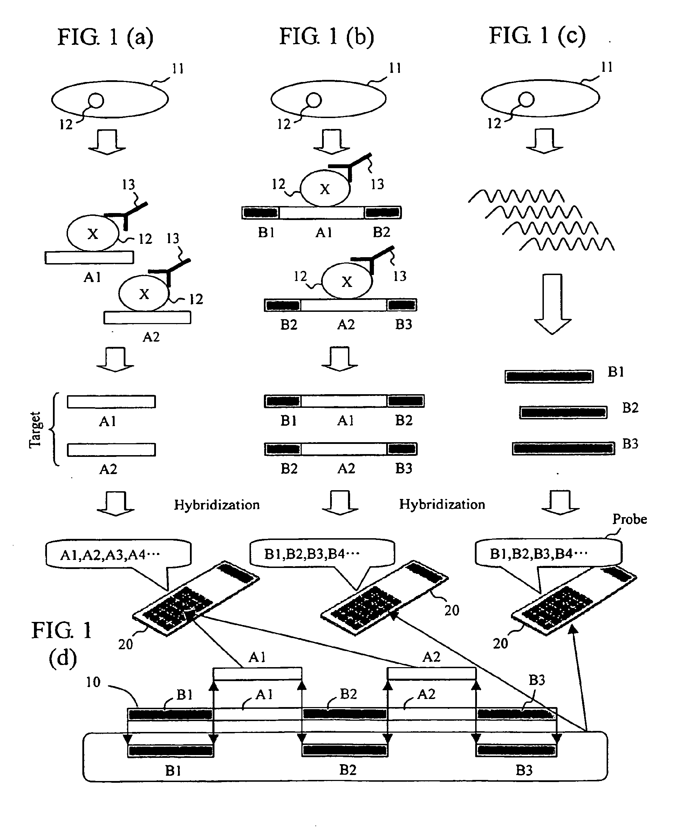

In the present example, fluorescent intensity data is acquired using two conventional methods. One method is the method disclosed in Patent Document 1 and is shown in Fig. 1a schematically. As shown in Fig. 1d, genomic DNA 10 is comprised of coding regions B1, B2, B3, ..., which are called exons, and noncoding regions A1, A2, A3, ..., which are called introns. In the following, coding regions will be simply referred to as genes, with the noncoding regions being referred to as intergenic regions as they appear.

As a probe, the noncoding regions A1, A2, and A3 of the genomic DNA are used. A DNA chip 20 is prepared by spotting the noncoding regions A1, A2, and A3 on a slide glass. Then, a DNA binding protein X12 from a specific organism 11 is bound to the genomic DNA by cross-linking. The genomic DNA is then disintegrated with an ultrasonic disintegrator. The resultant DNA fragments are extracted using an antibody 13 that specifically recognizes the protein X. Thereafter the cross-linking is removed, and the DNA binding protein X is separated from the DNA fragments. The thus separated DNA fragments are labeled with a fluorescent dye, thereby obtaining a target. The DNA fragments of the target include the noncoding regions A1, A2, and A3 that can bind to the protein X12. The target is then hybridized to the probe of the DNA chip, whereby fluorescent intensity data can be obtained. Based on this fluorescent intensity data, the binding site of the protein X is determined, as will be described below.

The other is the method disclosed in Patent Document 2 and is schematically shown in Fig. 1b. In this second method, coding regions B1, B2, and B3 of the genomic DNA are used as a probe. A DNA chip 20 is prepared by spotting the coding regions B1, B2, and B3 on a slide glass. The DNA fragments of the target include the noncoding regions A1, A2, and A3, and parts B and B2 of the coding regions on either side of the noncoding regions. The target is hybridized to the probe of the DNA chip, whereby fluorescent intensity data can be obtained. Based on this fluorescent intensity data, the binding regions of the DNA binding protein X are determined, as will be described below.

In the present example, a control test was also conducted, as shown in Fig. 1c. In the control test, a target was prepared without using the antibody that recognizes the specific protein X. The DNA fragments of the target consisted of the coding regions B1, B2, and B3. The target was hybridized to the coding regions, which are the probe of the chip, and then fluorescent intensity data was measured.

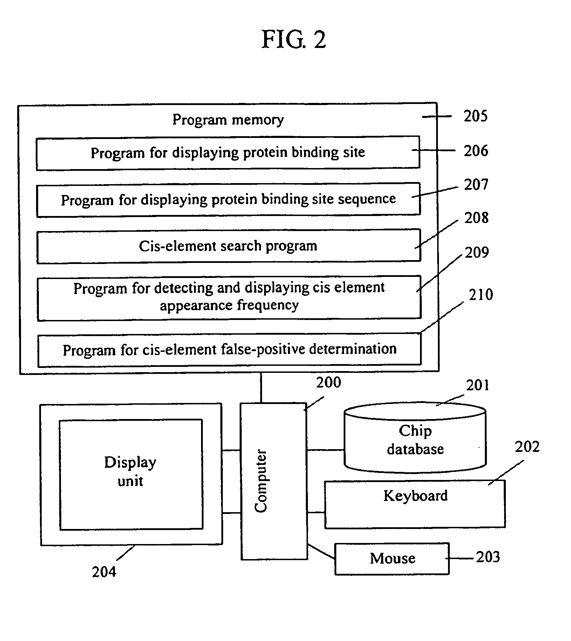

With reference to Fig. 2, an example of a protein binding site identification system of the invention is described. The system of the present example includes a computer 200, a chip database 201, a keyboard 202, a mouse 203, a display unit 204, and a program memory 205.

The chip database 201 stores fluorescent intensity (hybridization intensity) data obtained by the two methods described with reference to Fig. 1, and blank data obtained from the control test. The keyboard 202 and mouse 203 constitute the interface for the input and output of data. The display unit 204 displays the results of executing a variety of programs, as well as providing the interface for the input and output of data. The computer 200 processes the programs stored in the program memory 205.

The program memory 205 includes: a program 206 for displaying the protein binding site on the genomic DNA; a program 207 for displaying a list of the sequences of protein binding sites on the genomic DNA; a program 208 for retrieving cis elements and displaying them in a list; a program 209 for detecting and displaying the frequency of appearance of specific cis elements in a designated sequence; and a program 210 for determining whether a specific cis element is a false positive and displaying the result of determination.

The cis-element search program 208 utilizes MEME (Multiple EM for Motif Elicitation) based on a conventional EM algorithm. The cis-element false-positive determination program 210 determines a particular cis element to be a false positive if it appears in sequences with lower fluorescent intensity when cis elements appear in a plurality of sequences. A false positive herein means that the possibility of a specific protein specifically recognizing the cis element in the genome sequence is small. If a cis element appears in a single sequence a number of times, the program determines it to be a positive, thereby determining that the possibility of a specific protein controlling the sequence is high.

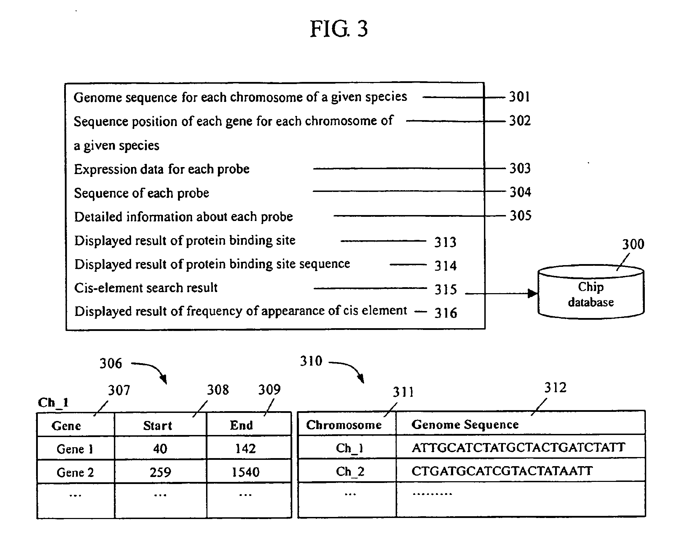

Fig. 3 shows the contents of data stored in the chip database 201. "Genome sequence for each chromosome of a given species" 301, which is the genome sequence of a target entered by the user, is a database in the form as shown in Table 310, for example. The table 310 contains a chromosome number 311 and a corresponding genome sequence 312. "Sequence location of each gene for each chromosome of a given species" 302, which shows the location of the genome sequence of a target entered by the user, is a database in the form of a Table 306, for example. Table 306 contains the gene number 307, start site of gene 308, and end site 309.

"Expression data about each probe" 303, which shows the fluorescent intensity (hybridization intensity) of each probe mounted on the DNA chip, is experimental data entered by the user. "Sequence of each probe" 304 is the base sequence of each probe, and it is experimental data entered by the user. "Detailed information about each probe" 305 is an annotation to a particular gene when the probe is a coding region. It is entered by the user as required.

"Displayed result of protein binding site" 313 shows the results of executing the program 206 for displaying protein-binding sites. "Displayed result of protein binding site sequence" 314 shows the result of executing the program 207 for displaying protein-binding site sequences. "Result of cis element search" 315 shows the result of executing the cis-element search program 208. "Displayed result of detecting the frequency of appearance of cis elements" 316 shows the result of executing the program 209 for detecting and displaying cis-element frequencies and that of the program 210 for determining cis-element false positives. The results of executing these programs are entered by the user as required.

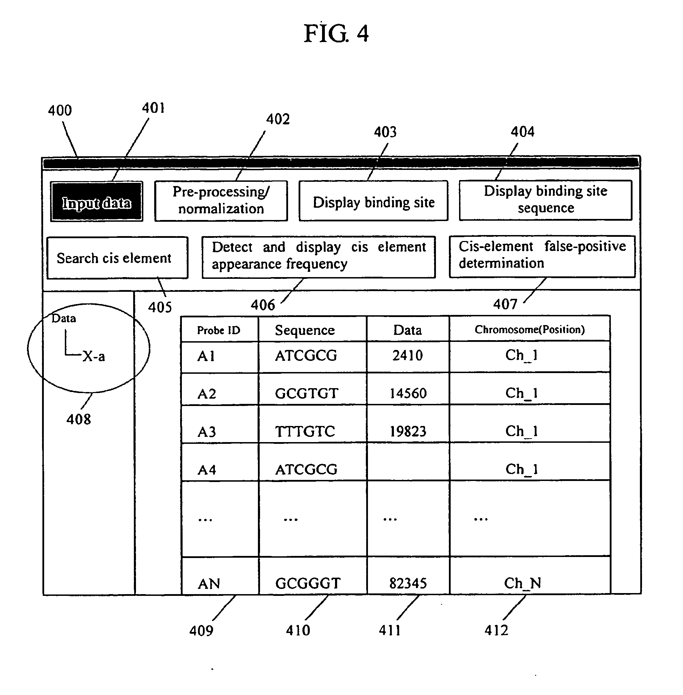

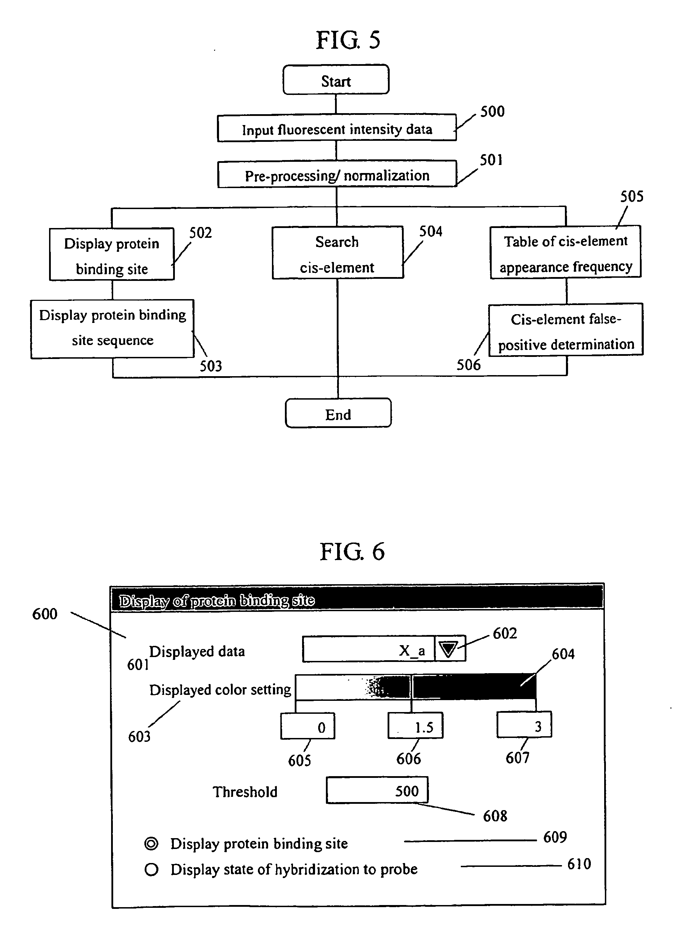

With reference to Figs. 4 and 5, the flow of the programs stored in the program memory 205 is described. Fig. 4 shows an example of a screen 400 displayed on the display unit 204 upon starting up of the system of the present example. At the top of the screen 400, tool buttons for running the various programs are shown. The tool buttons include a data input button 401; pre-processing and normalizing button 402; a button for displaying a binding site 403; a button 404 for displaying a binding site sequence; a button 405 for the retrieval of a cis element; a button 406 for detecting and displaying the frequency of appearance of a cis element; and a button 407 for determining cis-element false positives.

After the system of the example is started up, the user, at step 500, clicks the button 401 for data entry and enters fluorescent intensity data. The user further enters various data shown in Fig. 3. To the left of the screen 400, a shortcut 408 with a data tree is displayed to the right of the screen, lists 409 to 412 of input data are displayed. These lists include a list 409 of probe IDs uniquely representing each probe on the DNA chip, a list 410 of robe sequences, a list 411 of fluorescent intensity, and a list 412 of locations of probes on chromosome (genome sequence). At step 501, the user clicks the pre-processing and normalizing button 402, whereby the fluorescent intensity data entered is processed.

When the portions in the genome sequence where a given protein X has bound are to be visually displayed, the user clicks the binding site display button 403 at step 502, whereby the protein binding site display program 206 is executed. When a list of sequences in the genome sequence to which a given protein X has bound are to be displayed, the user, at step 503, clicks the binding site sequence display button 404, whereby the protein binding site sequence display program 207 is executed. Thus, the sites in the genome sequence where the protein X has bound can be specified.

When examining which cis element in the binding sites has been recognized by the protein X when it bound to the binding sites, the user clicks the cis element search button 405 at step 504. As a result, the cis element search program 208 is executed, and short sequences (cis elements) that appear commonly in the binding sites are retrieved.

Further, when the frequency of appearance of a specific cis element in the genome sequence is to be examined from the cis element search result, the user, at step 505, clicks the button 406 for detecting and displaying the frequency of appearance of cis element, whereby the cis element appearance frequency detecting and displaying program 209 is executed. When determining whether a specific cis element is a false positive, the user clicks the cis element false-positive determination button 407 at step 506. This causes the cis element false-positive determination program 210 to be executed, and a false-positive determination is made on the cis element.

Fig. 6 shows a screen 600 that is displayed when the user clicked the binding site displaying button 403 at step 502. When the binding site displaying button 403 is clicked, the protein binding site displaying program 206 is executed. The screen 600 shows a condition setting dialog for displaying a protein binding site. The condition setting dialog includes displayed data 601 and a pulldown menu 602. The user operates the pulldown menu 602 to display the list of data 408 that has already been entered. The user then selects displayed data from the displayed list of information.

At the bottom of the displayed data 601 and the pulldown menu 602, there is provided a displayed color setting 603 with a bar 604 for representing fluorescent intensity by the gradation of certain colors. At the bottom of the bar 604, there is shown a minimum value 605, an average value 606, and a maximum value 607, indicating the gradation in numerals. These values 605 to 607 are initially set to default average values determined from entered data. The user can change the hue by directly changing the values 605 to 607.

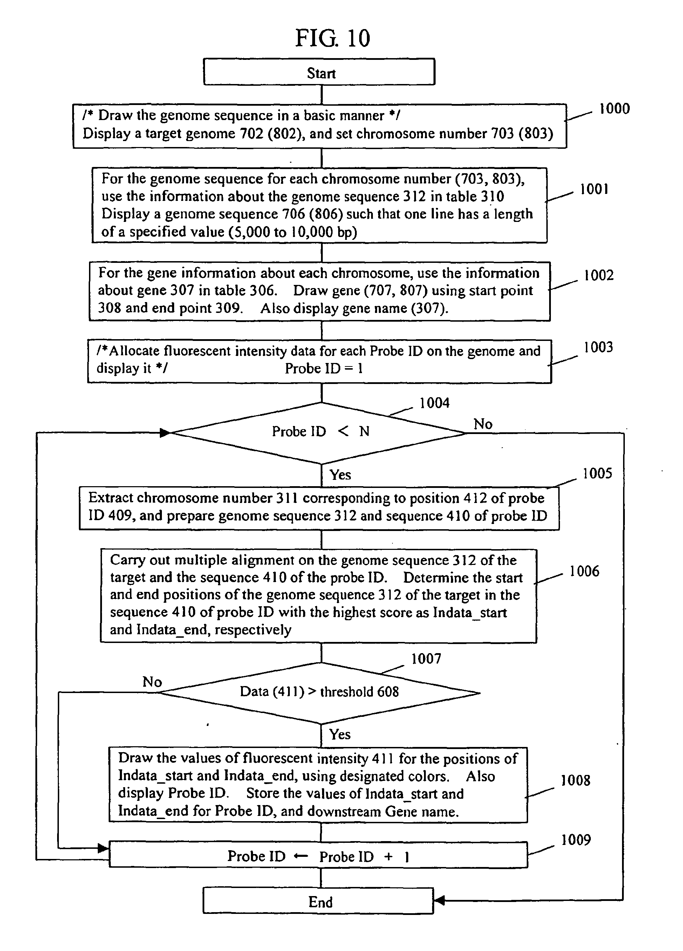

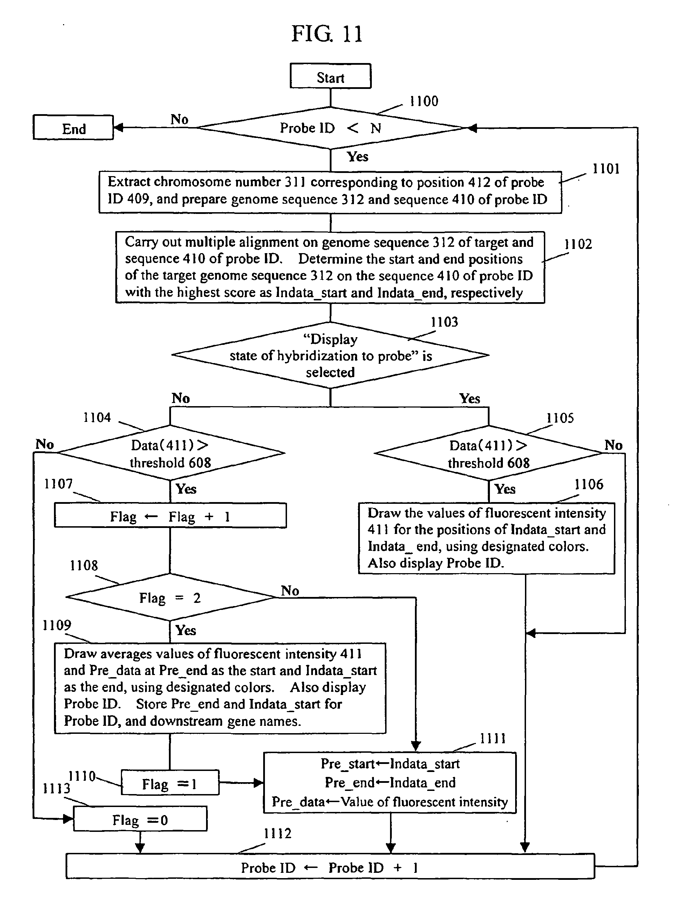

A threshold value 608 indicates the lower limit of fluorescent intensity beyond which it is determined that hybridization has not been fully conducted. If the fluorescent intensity is below the threshold, the particular probe ID is eliminated from the candidates for protein binding sites. Probe IDs with data that is below the threshold are excluded from color display during the processing of programs shown in Figs. 10 and 11.

The user can select either "Display protein binding site" 609 or "Display state of hybridization to probe" 610.

In the experiment shown in Fig. 1 a, involving the use of a noncoding-region probe, protein binding sites (noncoding regions) can be clarified by hybridization. Thus, the user selects "Display protein binding site" 609.

In the experiment shown in Fig. 1 b involving the use of coding region probe, because hybridization occurs in coding regions, fluorescent intensities do not directly represent protein binding sites (noncoding regions). Therefore, the user selects "Display state of hybridization to probe" 610. However, when the fluorescent intensity of an adjacent coding region exceeds the threshold value, "Display protein binding site" 609 may be selected. In this case, the noncoding regions with respect to the two coding regions with fluorescent intensity exceeding the threshold are displayed as protein binding sites.

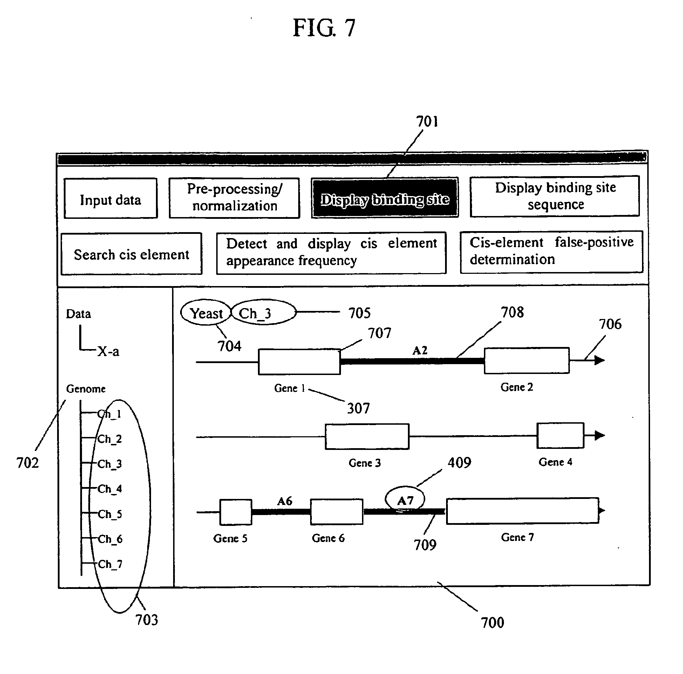

Fig. 7 shows a screen 700 that is displayed when the user has selected "Display protein binding site" on the screen of Fig. 6. To the left of the screen 700, there are displayed the genome 702 of the target in Table 310 of Fig. 3 and chromosome numbers 703 contained in the genome. To the right of the screen 700, there are displayed the species 704 of the target, chromosome number 705, and the genome sequence 706 of probe. The genome sequence 706 extends from left to right in the direction of 5' to 3'. Although Fig. 7 shows three lines of genome sequence, it should be noted that the right-hand end of the first line connects to the left-hand end of the second line, and the right-hand end of the second line connects to the left-hand end of the third line. When the entire genome sequence 706 of one probe cannot be shown in one screen, an interface and GUI may be provided for transition to the next screen.

The location of the coding region 707 is identified from gene number 307 of the target, and start site 380 and end site 309 of gene in Table 306 in Fig. 3, and the target gene number 307 is displayed at the location. Probe ID 409 of Fig. 4 is displayed in noncoding region 709. The value of fluorescent intensity 411 of Fig. 4 is compared with the threshold value 608 of Fig. 6. If it exceeds the threshold value, the noncoding regions 708 and 709 are displayed with the color that is set in the displayed color setting 603.

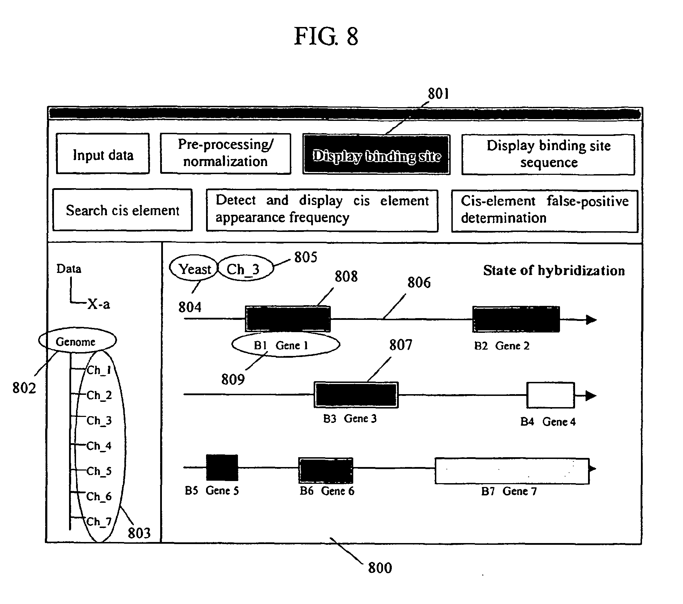

Fig. 8 shows a screen 800 that is displayed when "Display state of hybridization to probe" 610 on the screen has been selected by the user. As mentioned above, the user selects "Display state of hybridization to probe" 610 when the experiment of Fig. 1b has been performed.

To the left of the screen 800, there are displayed a genome 802 of the target in Table 310 of Fig. 3 and the chromosome number 803 included in the genome. To the right of the screen 800, there are displayed a species 804 of the target, chromosome number 805, and the genome sequence 806 of a probe. The genome sequence 806 is arranged from left to right in the direction of 5' to 3'. Although the genome sequence is displayed in three lines, the right-side end of the first line connects to the left-side end of the second line, and the right-side end of the second line continues onto the left-side end of the third line.

In the experiment shown in Fig. 1 b, because hybridization is conducted using coding regions as a probe, the locations of the coding regions 807 and 808 on the genome sequence of the probe are identified. At the bottom of the coding regions 807 and 808, coding region name and gene number of target 809 is displayed.

If the value of fluorescent intensity 411 in Fig. 4 exceeds the threshold value 608 shown in Fig. 6, the coding regions 807 and 808 are indicated with a color that has been set in the displayed color setting 603 of Fig. 6.

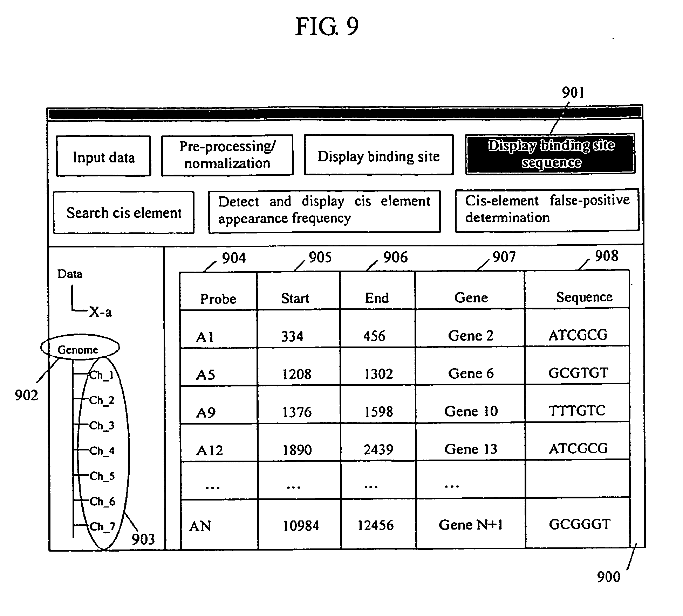

Fig. 9 shows a screen 900 that is displayed when a button 901 at the top of the tool buttons for displaying a binding site sequence is clicked. To the left of the screen 900, there is displayed a target genome 902 in Table 310 of Fig. 3 and the chromosome numbers 903 contained in the genome. To the right of screen 900, there is displayed the information about "Displayed result of protein binding site" 313 of Fig. 3. When the experiment of Fig. 1a has been conducted, the noncoding regions where protein has bound are displayed. There are also displayed probe ID 904, and the start site 905 and end site 906 of the sequence of the probe, namely, the binding sites. Further, name 907 of gene downstream of noncoding region, and the base sequence 908 on the chromosome from start site 905 to end site 906 are displayed.

Fig. 10 shows the flow of processes performed by the protein binding site displaying program 206 in the case where fluorescent intensity data has been obtained from the experiment shown in Fig. 1 a. At step 1000, the target genome 702 is displayed on the screen 700 of Fig. 7. Chromosome number 311 in Table 310 of Fig. 3 is extracted, and then chromosome number 703 is set. At step 1001, the information about genome sequence 312 with respect to the chromosome 311 in Table 310 is used for the genome sequence of each chromosome number 703. The genome sequence 706 is displayed such that each line has a length of a specified value, such as from 5000 to 10,000 bp. At step 1002, the information about gene 307 in Table 306 is used for the gene information about each chromosome. Based on the start point 308 and end point 309 in Table 306 of Fig. 3, gene 707 is drawn. Beneath gene 707, gene name 307 is also shown.

At step 1003, fluorescent intensities are allocated on the genome for each probe ID when they are displayed. For this purpose, a variable called Probe ID is set, the locations of 1^{st} to N-th data are determined, threshold determination is made based on the fluorescent intensities, and the fluorescent intensities are displayed with the designated colors. The ID of the probe determined to be a protein binding site is stored in the data region 313 of Fig. 3, together with its location information and the downstream gene name. Probe ID is then set to be 1.

At step 1004, it is determined whether the number in the variable Probe ID is smaller than the total number N of items of data for Probe ID. If it is smaller than N, the routine proceeds to the next step 1005.

At step 1005, chromosome number 311 corresponding to the position 412 on the genome sequence of the probe with Probe ID 409 is acquired from Table 310, and a corresponding genome sequence 312 and the sequence 410 of the corresponding probe ID are prepared. At step 1006, a multiple alignment program is run on the target genome sequence 312 and the sequence 410 of the probe ID. The locations in the sequence 410 of the probe ID where the genome sequence 312 of the target starts and ends with the highest score are determined as Indata_start and Indata end, respectively.

At step 1007, it is determined whether the fluorescent intensity 411 for the Probe ID is greater than the threshold value 608. If the fluorescent intensity 411 exceeds the threshold value 608, the routine proceeds to step 1008.

At step 1008, the values of the fluorescent intensity 411 for the locations of Indata_start and Indata_end are drawn with designated colors. The Probe ID is also displayed. The values of Indata_start and Indata_end for the Probe ID, and the downstream gene name are stored in the data 313 of Fig. 3. If the fluorescent intensity 411 did not exceed the threshold value 608, or if it exceeded the threshold and when the drawing and storing processes are completed, the same processes are repeated for the next probe.

Fig. 11 shows the flow of processes of the protein binding site display program 206 in a case where fluorescent intensity data has been obtained from the experiment of Fig. 1b. Step 1000 through step 1003 shown in Fig. 10 are performed in the same manner. After step 1003, steps 1100 to 1112 shown in Fig. 11 are performed, as described below.

At step 1100, it is determined whether the number in Probe ID of the probe to be processed is smaller than the total number N of Probe IDs. If it is smaller than N, the routine proceeds to next step 1101.

At step 1101, for the chromosome number 311 at the location 412 on the genome sequence of the probe corresponding to the Probe ID 409, a genome sequence 312 and sequence information 410 for the probe ID are prepared. At step 1102, a multiple alignment program is run on the sequence information 410 of the probe ID for the target genome sequence 312. The start site and end site of the sequence information 410 of the probe ID on the genome sequence 312 that have the highest scores are determined as Indata_start and Indata_end, respectively.

At step 1103, the user determines whether "Display protein binding site" 609 or "Display state of hybridization to probe" 610 in Fig. 6 has been selected. If "Display protein binding site" 609 has been selected, the routine proceeds to step 1104. If "Display state of hybridization to probe" 610 has been selected, the routine proceeds to step 1105.

At step 1105, it is determined if the fluorescent intensity 411 is greater than the threshold value 608. If the fluorescent intensity 411 is greater than the threshold value 608, the routine proceeds to step 1106. If the fluorescent intensity 411 is not greater than the threshold value 608, the routine proceeds to step 1112.

At step 1106, the values of the fluorescent intensity 411 for the locations of Indata_start and Indata_end are drawn with designated colors. Probe ID is also displayed.

At step 1104, it is determined whether or not the fluorescent intensity 411 is greater than the threshold value 608. If the fluorescent intensity 411 is greater than the threshold value 608, the routine proceeds to step 1107. If not, the routine proceeds to step 1103 where Flag is set to zero.

At step 1107, 1 is added to the variable Flag. If Probe ID is 1, 1 is entered in Flag. At step 1108, it is determined whether Flag is 2. If not, the value of Indata_start is set in a variable Pre_start at step 1111, the value of lndata_start is set in a variable Pre_end, and the value of the fluorescent intensity 411 is set in Pre_data.

If Flag is 2, which means that the fluorescent intensity of hybridization of a previous Probe ID has exceeded the threshold value and the fluorescent intensity of the next Probe ID has also exceeded the threshold value, the routine proceeds to step 1109. At step 1109, Pre_end is considered to be the start and Indata_start is considered to be the end, and the average values of the fluorescent intensity 411 and Pre_data are drawn at these positions, respectively, with designated colors. Probe ID is also displayed. Pre_end and Indata_start for Probe ID, and downstream gene names are stored.

At step 1110, Flag is set to be 1 and the routine proceeds to step 1111 where Indata_start and Indata_end for the current Probe ID are set in Pre_start and Pre_end, respectively. The value of the fluorescent intensity 411 is also set in Pre_data. At step 1112, the next Probe ID is processed by repeating the same sequence from the initial step 1101.

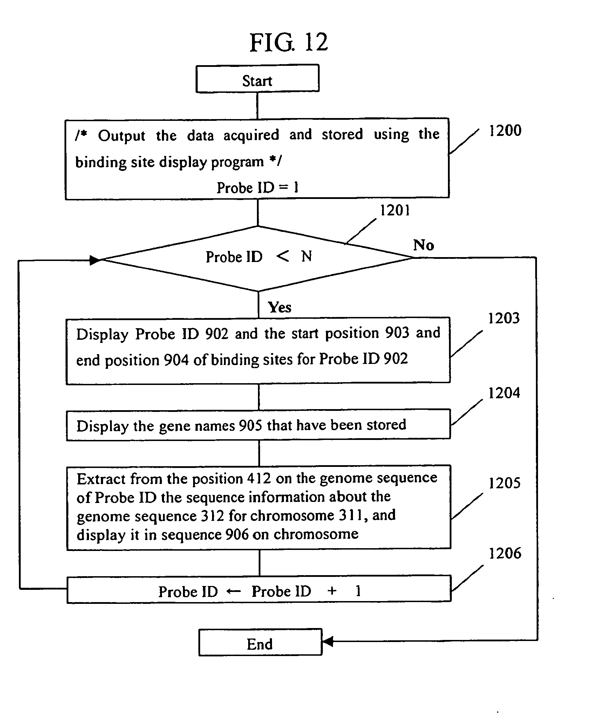

Fig. 12 shows the flow of processes performed by the program 207 for displaying protein binding site sequence. At step 1200, using the data stored at step 1008 of Fig. 10 or step 1109 of Fig. 11, the list of chromosome numbers at the left of the screen 900 of Fig. 9 is displayed. Probe ID is initially set to be 1.

At step 1201, it is determined if the number in Probe ID of the probe to be processed is smaller than the total number N of Probe IDs. If smaller than N, the routine proceeds to the next step 1203.

At step 1203, the probe ID 904 on the screen 900, and the start site 905 and end site 906 of binding sites for Probe ID 904 are displayed. At step 1204, gene name 907 is displayed. At step 1205, based on the location 412 on the genome sequence of Probe ID, the sequence information for the genome sequence for the chromosome 311 is acquired, and the base sequence 908 on the chromosome is displayed. At step 1206, the same steps are repeated for the next Probe ID.

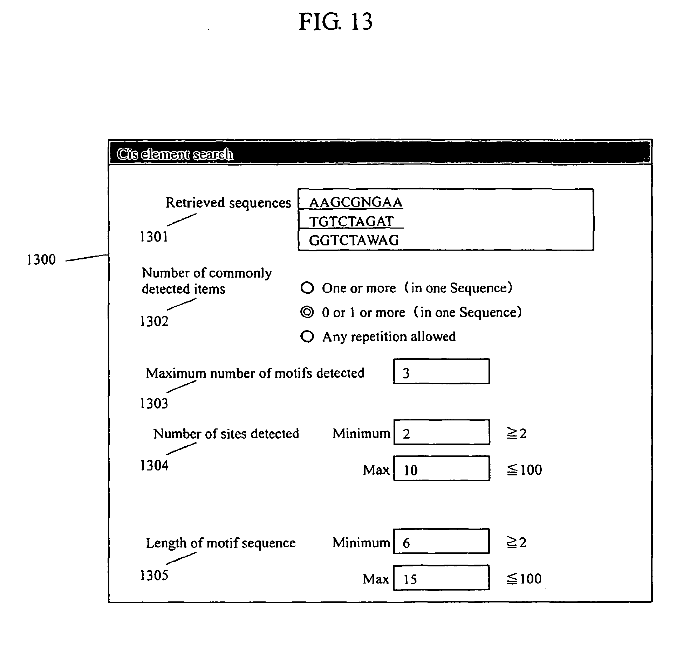

Fig. 13 shows a screen 1300 that is displayed when the user has clicked the cis element search button 405. When the cis element search button 405 is clicked, the cis element search program 208 is run. The screen 1300 shows a condition setting dialog for cis element search. In Searched sequence 1301, sequences from which cis elements (motif sequences) are to be retrieved are entered. For example, a plurality of sequences upstream of gene are entered. It is also possible to extract motif sequences using upstream sequences of a specific gene group from experimental data obtained with a conventional DNA chip. This option may be selected when the gene name 907 downstream of a binding site and its sequence 908 are shown in the screen 900 of Fig. 9.

In "Number of commonly detected items" 1302, three alternatives are shown. One is when one or more cis elements (motif sequences) are detected from each searched sequence. Another is when zero or one or more cis elements (motif sequences) are detected. The other is when any repetition is allowed. In "Maximum number of motif detection" 1303, the maximum number of cis elements (motif sequences) to be detected commonly from all of the searcged sequences is designated. In "Number of detected sites" 1304, the number of cis elements (motif sequences) to be detected from a single searched sequence is designated. "Length of motif sequence" 1305 is the item for designating the length of retrieved cis elements. The values that can be set in "Number of detected sites" 1304 and "Length of motif sequence" 1305 are from 2 to 100. If any other values are entered, an error message is displayed.

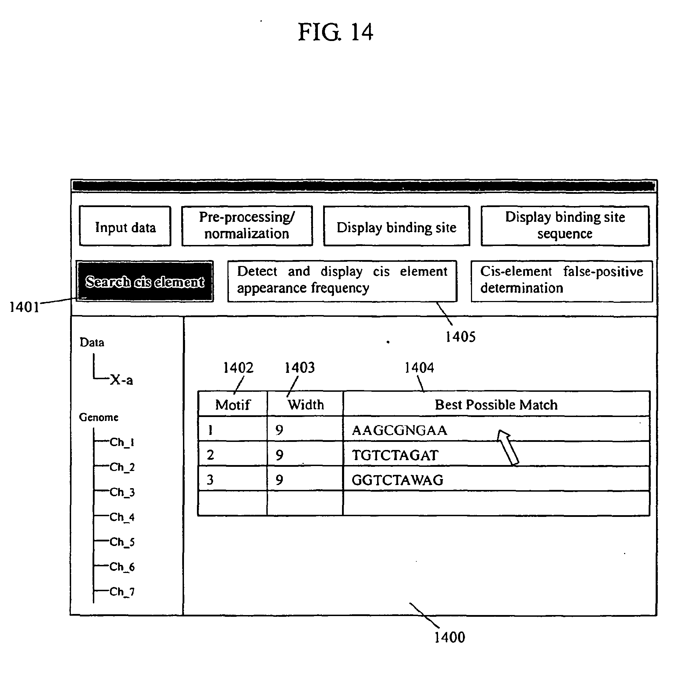

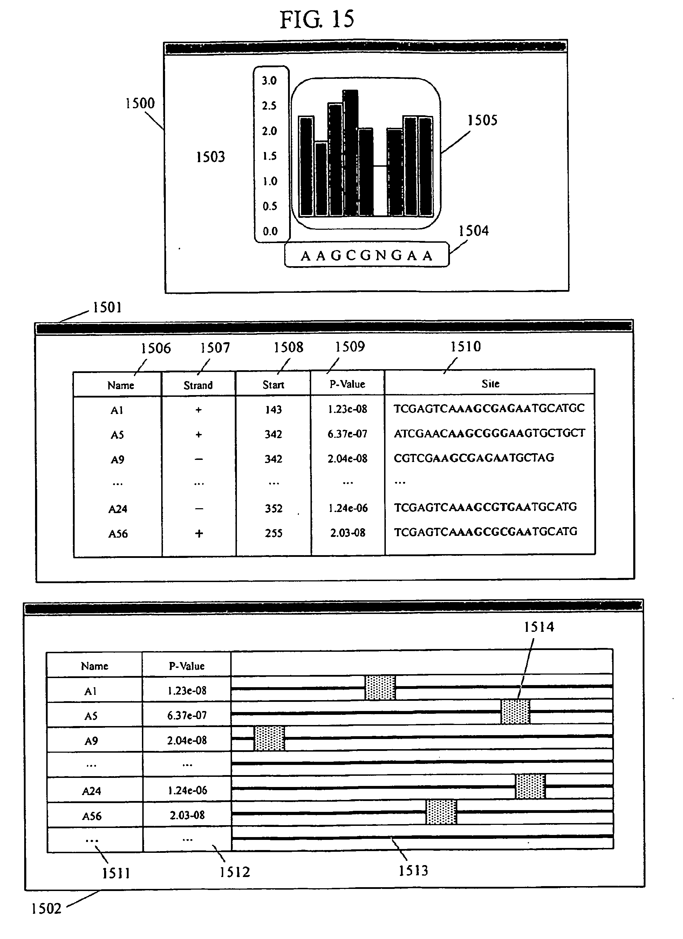

Fig. 14 shows a screen 1400, which is displayed when the entries on the screen 1300 of Fig. 13 are completed, shows cis element search results, which include motif number 1402, length of motif sequence 1403, and motif sequence 1404. When one line is selected from this list and clicked, the details of the information contained in the line are displayed in a separate window, as shown in Fig. 15.

Fig. 15 shows an example where detailed data about a particular portion of the cis element search results displayed on the screen 1400 of Fig. 14 are displayed in terms of three images. For the program for the retrieval of cis elements, MEME utilizing an EM algorithm is employed. Use of the algorithm allows short motif sequences commonly contained in multiple items of sequence data to be statistically extracted. The detailed results are provided in the same way as the display method compatible with the MEME.

A screen 1500 shows a bar graph 1505 of the frequency of appearance of the base sequence of a motif sequence. The horizontal axis 1504 shows base sequence, and the vertical axis 1503 shows E-values (expected values).

A screen 1501 shows a table of the locations of motif sequences in the searched sequence. The table includes probe ID 1506 uniquely indicating a searched sequence, strand 1507 indicating the direction of motif sequence, start site 1508 of motif sequence in the searched sequence, P-value (significance probability) 1509 of the motif, and the motif sequence 1510 with 10 previous and subsequent bases. The + sign in strand 1507 indicates the direction from 5' to 3', and the - sign indicates the opposite direction.

A screen 1502, which visually represents the location of a motif sequence in the searched sequence, is basically the same as the screen 1501 in terms of contents. The screen 1502 includes probe ID 1511, P-value of motif 1512, searched sequence 1513, and motif sequence 1514.

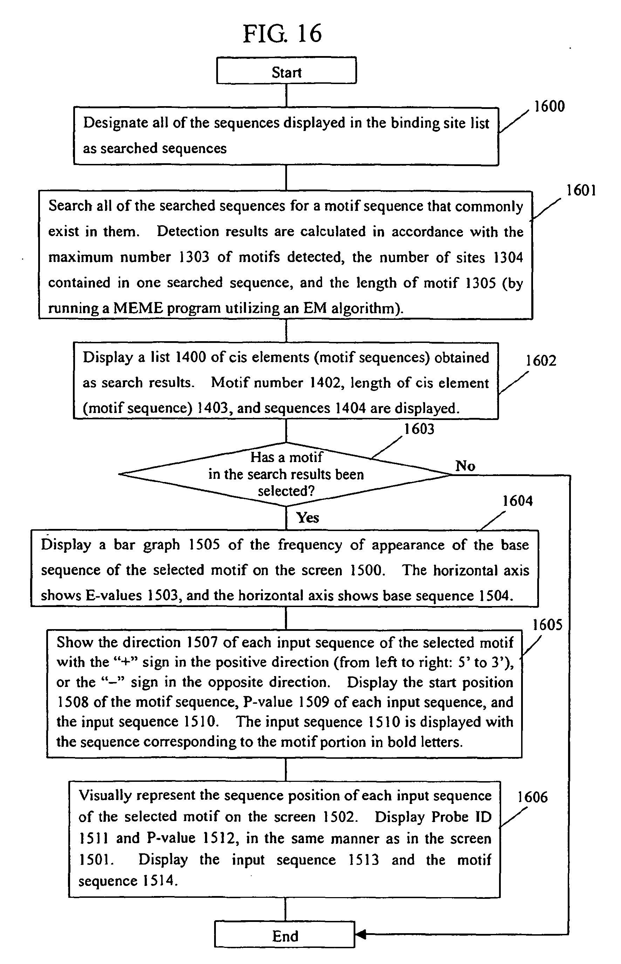

Fig. 16 shows the outline of the processes performed by the cis element search program 208. As mentioned above, the cis element search program 208 is compatible with a MEME program. At step 1600, the searched sequence for the retrieval of a motif sequence is designated. In the present example, all of the sequences in the binding site list displayed on the screen 900 of Fig. 9 are used as searched sequences. Alternatively, the searched sequences entered in "Searched sequence" 1301 on the screen 1300 of Fig. 3 may be used as the searched sequences. Thereafter, at step 1601, a motif sequence commonly contained in all of the searched sequences is retrieved. Search conditions include, as designated on the screen 1300 of Fig. 13, the maximum number of detected motif 1303, the number of detected sites 1304 contained in each searched sequence, and the length of motif sequence 1305, for example.

At step 1602, the cis elements obtained as search results are displayed. Specifically, motif number 1402, length of motif sequence 1403, and the sequence of cis element as the motif are displayed, as shown in Fig. 14. At step 1603, it is determined if the user has selected a specific motif sequence. If the user has selected a specific motif sequence, the information about the motif sequence selected by the user is displayed.

At step 1604, the screen 1500 of Fig. 15 is displayed. At step 1605, the screen 1501 of Fig. 15 is displayed. At step 1606, the screen 1502 of Fig. 15 is displayed.

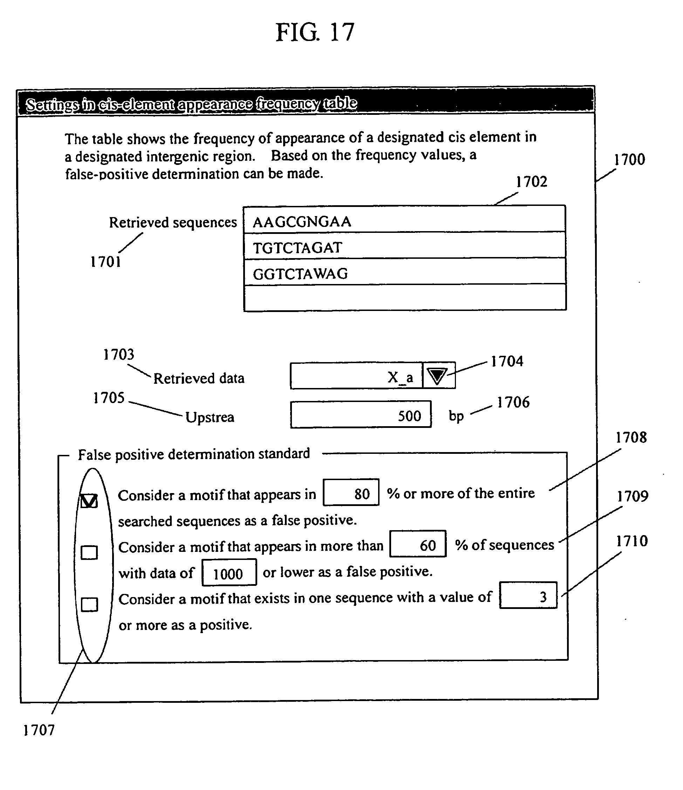

Fig. 17 shows a screen 1700 that is displayed when the user clicked the button 1405 on the screen 1400 of Fig. 14 for detecting and displaying the frequency of appearance of a cis element. When the button 1405 for detecting and displaying the frequency of appearance of a cis element is clicked, the program 209 for detecting and displaying the frequency of appearance of a cis element is executed. The screen 1700 shows a dialog for setting conditions for detecting cis element frequency and determining false positives.

"Retrieved sequences" 1701 shows the motif sequences (cis elements) of which the frequency of appearance is to be detected. When the screen 1700 is displayed, the list of motif sequences 1401 on the screen 1400 of Fig. 14 is automatically displayed. Alternatively, the user may select and enter a plurality of lines from the list of motif sequences 1404 on the screen 1400 of Fig. 14. The screen 1700 includes "Retrieved data" 1703 and a pulldown menu 1704. By operating the pulldown menu 1704, the user can cause data relevant to a search to be extracted from data that has already been entered and displayed. "Upstream" 1705 is an item for designating base pairs upstream of a particular binding site of gene. When "500" is entered in the item, 500 upstream base pairs are selected as searched objects.

At the bottom of the screen 1700, there is displayed a dialog for entering a false-positive determination standard. When a false-positive determination is to be made, one of three standards is designated. A first standard 1708 is where a motif sequence that appeared more often than a designated proportion with respect to the entire retrieved sequences is considered a false positive. A second standard 1709 is where a motif sequence that appeared in sequences with data that is below a designated value more often than a designated proportion value is considered to be a false positive. A third standard 1710 is such that a motif sequence that appeared in a single sequence more often than a designated proportion value is considered a positive.

In accordance with the first standard, a motif sequence that appeared in more than 80% with respect to the upstream of the entire searched genes has too high a frequency. Because such a sequence is difficult to be considered a sequence which a protein X would specifically recognize, it is considered to be a false positive. In accordance with the second standard, a motif sequence that was contained in the data with lower fluorescent intensities is difficult to be considered to be a sequence which the protein X would specifically recognize. Therefore, such a sequence is considered to be a false positive. In accordance with the third standard, if a motif sequence exists in one upstream sequence with a high frequency, the possibility of the motif sequence being controlled by the protein X is high, and therefore the sequence is considered to be a positive.

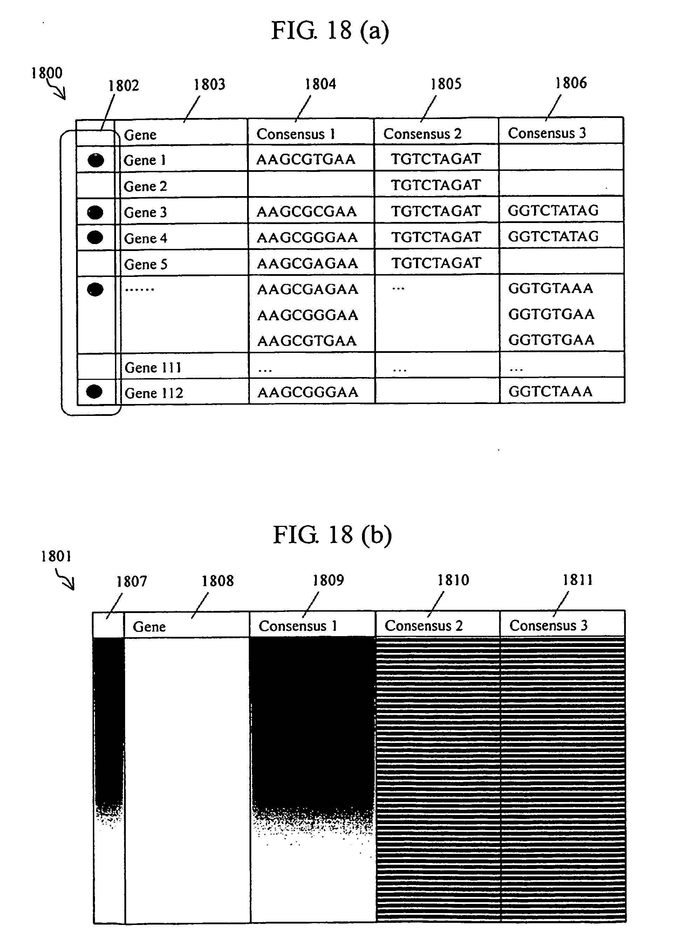

Fig. 18a shows an example of the table 1800 of frequency of appearance of cis elements obtained by running the program for detecting and displaying the frequency of appearance of cis elements. The table 1800 of cis element appearance frequencies include gene names 1803 and consensus sequences 1804 to 1806. At the head 1802 of each of the gene names 1803, a mark is indicated if the gene corresponds to an upstream location of a gene shown in the image 900 shown in Fig. 9 that has been determined to be a protein binding site. When a mark is indicated, this shows that the motif sequence is likely to control the gene shown in a particular item in gene names 1803. The consensus sequences 1804 to 1806 indicate whether the motif sequence designated in "Retrieved sequence" 1701 in the screen 1700 of Fig. 17 is contained in an upstream region of each gene with gene name 1803. As shown, when there are a plurality of sequences designated in an upstream region of each gene with gene name 1803, they are displayed. When the motif sequences (cis elements) are designated in accordance with the IUPAC notational system (International Union of Pure and Applied Chemistry), ambiguous portions may be shown with bold letters. In this way, the binding sites in retrieved data and the frequency of appearance of binding sequences can be recognized at once.

The entire lines of the table 1800 of cis element appearance frequencies can be sorted in either ascending or descending order. For instance, when an ascending sort is carried out with respect to the marks 1802, a false-positive determination table 1801 can be obtained, as shown in Fig. 18b. The first column 1807 of the false-positive determination table 1801 indicates the distribution of the marks 1802. The color densities schematically represent the density of the marks.

The first column 1807 is shown darker towards the top and lighter towards the bottom, indicating that the binding sites for the protein X are concentrated at the top of the false-positive determination chart 1801. When the second standard 1710 of Fig. 17 is set as the standard for the determination of false positives, a cell representing a specific gene upstream region is displayed in blue. A line 1809 of consensus 1 is shown in darker shades of blue towards the top and in lighter shades of blue towards the bottom. Lines 1810 and 1811 of consensus 2 and 3, respectively, are shown in uniform shade of blue throughout from top to bottom. Thus, it can be seen that consensus 1 is more likely than consensus 2 or 3 to be a cis element that the protein X specifically recognizes.

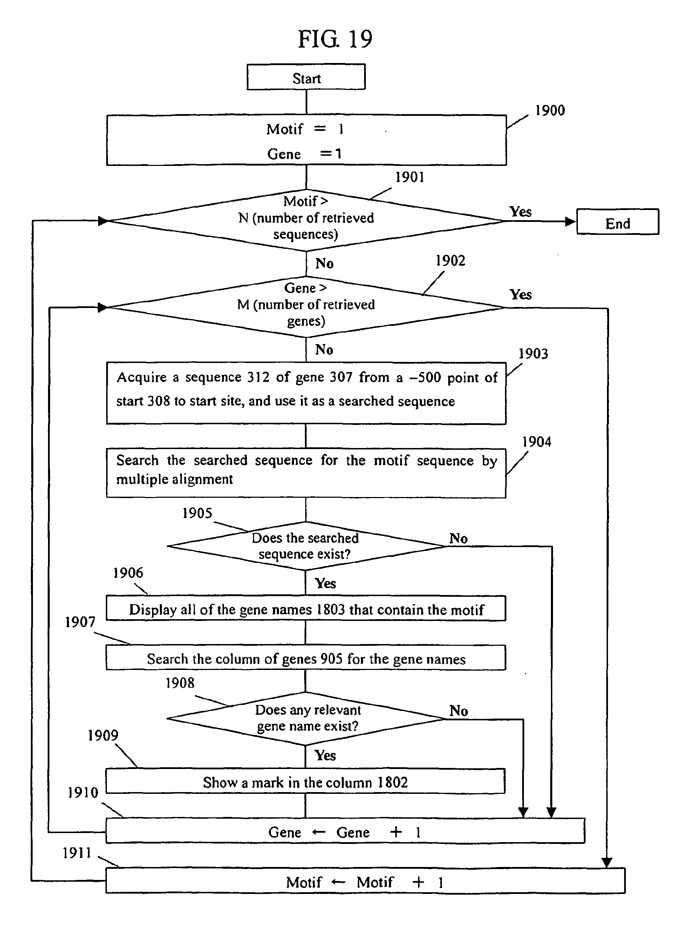

Fig. 19 shows the outline of processes performed by the program for detecting and displaying the frequency of appearance of cis element. The program detects at what frequency a particular motif sequence (cis element) appears in upstream sequences of all genes, in accordance with the conditions set on the screen 1700 of Fig. 17. At step 1900, initial settings are made such that Motif = 1 and Gene = 1. "Motif = 1" represents the first sequence in "Retrieved sequences" 1701 on the screen 1700 of Fig. 17. "Gene = 1" represents the first one of the genes in "Retrieved data" 1704. At step 1901, it is determined if Motif is greater than N. If Motif is greater than N, the program terminates. If Motif is not greater than N, the routine proceeds to step 1902. N is the number of sequences set in "Retrieved sequences" 1701. At step 1902, it is determined if Gene is greater than M. If Gene is greater than M, the routine proceeds to step 1911 where the next sequence is processed. If Gene is not greater than M, the routine proceeds to step 1903.

At step 1903, the sequence of Gene 307 between a location that is 500 bases prior to Start 308 and Start is acquired from the genome sequence 312 in the chip database 201 of Fig. 3. The thus acquired sequence is used as a retrieved sequence. At step 1904, a multiple alignment is conducted, and it is determined in step 1905 if the Motif sequence is contained in the searched sequence. If the motif sequence is contained, the routine proceeds to step 1906. If not, the routine continues onto step 1910 where the next Gene 307 is processed.

At step 1906, all of the gene names 1803 that contain the motif sequence are displayed. At step 1907, the column 907 of gene names downstream of binding sites in the screen 900 of Fig. 9 is searched for the names of genes 1803 that contain the motif sequence. If a gene name or names that contain the motif sequence exist in the gene names 907 of gene downstream of binding sites, the routine proceeds to step 1909. If there is no such gene name, the routine proceeds to step 1910 where the next gene is processed. At step 1909, a mark is indicated in the column 1802 at the left of the corresponding gene.

The processes from steps 1903 to 1909 are carried out on all of the genes in the retrieved data 1704. Further, these processes are carried out for all of the motif sequences in the retrieved sequences 1701.

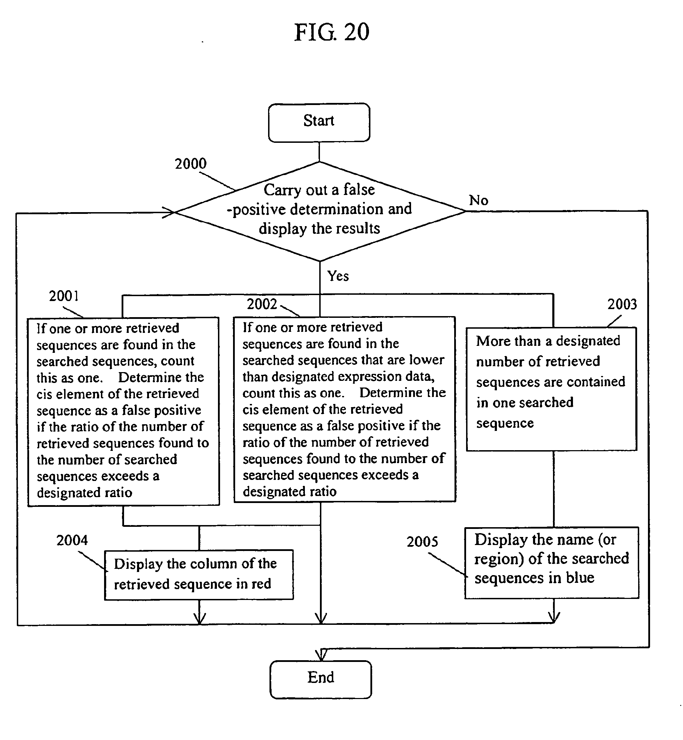

Fig. 20 shows the outline of the processes performed by the program for determining false positives. At step 2000, it is determined if any of the first to third standards for the determination of false positives on the screen 1700 of Fig. 17 has been selected. Namely, it is determined if there is any check mark 1707 on the screen. If none of the false-positive determination standards are set, the program terminates.

At step 2001, if one or more retrieved sequences are found in the searched sequences, this counts as one, and when the ratio of the number of retrieved sequences found to the number of searched sequences exceeds a designated value, the retrieved cis element is determined to be a false positive.

At step 2002, if one or more retrieved sequences are found in searched sequences that are lower than the designated expression data, this counts as one, and when the ratio of the number of such retrieved sequences to the number of searched sequences exceeds a specified value, the retrieved sequences are determined to be false positives. At step 2004, the sequences determined to be false positives are shown in red.

At step 2003, if more than a specified number of retrieved sequences are contained in a single searched sequence, the retrieved sequences are determined to be positives. At step 2005, the sequences determined to be positives are shown in blue.

## Claims

1. A method for identifying a protein binding site on a genomic DNA, comprising:
preparing a DNA fragment as a probe to be spotted on a DNA chip comprising a gene-noncoding region on said genomic DNA, preparing a fluorescently labelled DNA fragment as a target comprising a gene-noncoding region on genomic DNA, to which DNA fragment a specific protein is bound by cross-linking, extracting said DNA-fragment using an antibody specifically recognizing said specific protein, removing said cross-linking, separating said specific protein from said DNA fragment, labelling said DNA fragment with a fluorescent dye, thereby obtaining a target and causing said target to be hybridized to said probe, thereby obtaining first fluorescent intensity data;
preparing a DNA fragment as a probe to be spotted on said DNA chip comprising a gene coding region on said genomic DNA, preparing a fluorescently labelled DNA fragment as a target comprising a gene-noncoding region and part of coding regions on either side thereof on DNA, to which DNA fragment a specific protein is bound by cross-linking, extracting said DNA-fragment using an antibody specifically recognizing said specific protein, removing said cross-linking, separating said specific protein from said DNA fragment, labelling said DNA fragment with a fluorescent dye, thereby obtaining a target and then causing said target to be hybridized to said probe, thereby obtaining second fluorescent intensity data;
conducting a control test without using an antibody specifically recognizing said specific protein, by preparing a DNA fragment as a probe to be spotted on said DNA chip comprising a gene coding region on said genomic DNA, preparing a fluorescently labelled DNA fragment as a target comprising a coding region, causing said target to be hybridized to said probe, thereby obtaining fluorescent intensity data from said control test;
entering said first and said second fluorescent intensity data and information about said probes and said targets;
detecting a binding site on said genomic DNA of said probes to which said specific protein binds, based on said first and said second fluorescent intensity data and said information about said probes and said targets, and then visually displaying said binding site on the genome sequence of said probes;
detecting a candidate for a cis element on said genomic DNA to which said specific protein binds; and
detecting the frequency of appearance of said cis element in a gene-noncoding region on said genomic DNA.

2. The method for identifying a protein binding site according to claim 1, further comprising displaying a list of the base sequences of gene-noncoding regions on said genomic DNA to which said specific protein binds.

3. The method for identifying a protein binding site according to claim 1 or 2, wherein the binding site displaying step comprises:
carrying out multiple alignment on the sequence of said probe with respect to the genome sequence of said target, using said first fluorescent intensity data, so as to identify the location of the genome sequence of said target on the sequence of said probe;
comparing the fluorescent intensity of the sequence of said probe at the thus identified location with a threshold value; and
visually displaying, if said fluorescent intensity is greater than said threshold value, the noncoding region of the sequence of said probe with a specific color.

4. The method for identifying a protein binding site according to claim 1 or 2, wherein the binding site displaying step comprises:
carrying out multiple alignment on the sequence of said probe with respect to the genome sequence of said target, using said second fluorescent intensity data, so as to identify the location of said genome sequence of said target on the sequence of said probe;
comparing the fluorescent intensity of the sequence of said probe at the thus identified location with a threshold value; and
visually displaying, if said fluorescent intensity is greater than said threshold value, the coding regions on either side of the noncoding region of the sequence of said probe with a specific color.

5. The method for identifying a protein binding site according to one of the preceding claims, wherein said information about said probes that is entered in the data entry step comprises the base sequence of said probes and positional information about said probes on said genomic DNA, and wherein said information about said targets comprises the genome sequence of each chromosome of the species used in a relevant experiment, and the location of a gene coding region for each chromosome.

6. The method for identifying a protein binding site according to one of the preceding claims, wherein the cis element frequency detecting step comprises retrieving, using MEME (Multiple EM for Motif Elicitation) based on an EM algorithm, cis elements that satisfy certain conditions, said conditions including the maximum number of cis elements retrieved, their length, and the number of sites contained in each retrieved sequence.

7. The method for identifying a protein binding site according to one of the preceding claims, further comprising making a false-positive determination on each cis element based on the frequency of appearance of specific cis elements obtained in the cis element frequency detecting step.

8. The method for identifying a protein binding site according to claim 7, wherein the false-positive determination step comprises:
detecting sequences from searched sequences that contain one or more cis elements that are to be retrieved, and determining a particular cis element to be a false positive if the number of the sequences detected exceeds a designated ratio with respect to the searched sequences;
detecting sequences from searched sequences with fluorescent intensities lower than a designated fluorescent intensity that contain one or more cis elements that are to be retrieved, and determining a particular cis element to be a false positive if the number of the detected sequences exceeds a designated ratio with respect to the searched sequences; and
determining a particular cis element to be a positive if the number of cis elements to be retrieved from a single search sequence exceeds a designated number, thus determining that the sequence is controlled by said specific protein.

9. The method for identifying a protein binding site according to claim 7 or 8, wherein the false-positive determination step comprises displaying said cis element determined to be a false positive with a color different from the color with which the cis element determined to be a positive is displayed.

10. The method for identifying a protein binding site according to one of claims 7 to 9, wherein the false-positive determination steps comprises creating a table of gene names and frequencies of appearance of cis elements that contain searched sequences contained upstream of genes, wherein individual columns in said table indicate, upon sorting in either ascending or descending order, the result of determination of control genes and false-positive determination regarding cis elements, using color gradations.

## Patentansprüche

1. Verfahren zum Identifizieren einer Proteinbindungsstelle auf einer genomischen DNA, umfassend:
Herstellen eines DNA-Fragments als eine auf einem DNA-Chip in Spots aufzutragende Sonde ("probe to be spotted"), umfassend eine Gen-nicht-codierende Region auf der genomischen DNA, Herstellen eines fluoreszenzmarkierten DNA-Fragments als ein Ziel ("target"), umfassend eine Gen-nicht-codierende Region auf genomischer DNA, wobei an das DNA-Fragment ein spezifisches Protein durch Quervernetzung gebunden ist, Extrahieren des DNA-Fragments unter Verwendung eines Antikörpers, der das spezifische Protein spezifisch erkennt, Entfernen der Quervernetzung, Trennen des spezifischen Proteins von dem DNA-Fragment, Markieren des DNA-Fragments mit einem Fluoreszenzfarbstoff, wodurch ein Ziel erhalten wird, und Hybridisierenlassen des Ziels an die Sonde, wodurch erste Fluoreszenzintensitätsdaten erhalten werden;
Herstellen eines DNA-Fragments als eine auf dem DNA-Chip in Spots aufzutragende Sonde, umfassend eine Gen-codierende Region auf der genomischen DNA, Herstellen eines fluoreszenzmarkierten DNA-Fragments als ein Ziel, umfassend eine Gen-nicht-codierende Region und einen Teil von codierenden Regionen auf beiden Seiten davon auf der DNA, wobei an das DNA-Fragment ein spezifisches Protein durch Quervernetzung gebunden ist, Extrahieren des DNA-Fragments unter Verwendung eines Antikörpers, der das spezifische Protein spezifisch erkennt, Entfernen der Quervernetzung, Trennen des spezifischen Proteins von dem DNA-Fragment, Markieren des DNA-Fragments mit einem Fluoreszenzfarbstoff, wodurch ein Ziel erhalten wird, und dann Hybridisierenlassen des Ziels an die Sonde, wodurch zweite Fluoreszenzintensitätsdaten erhalten werden;
Durchführung eines Kontrolltests ohne Verwendung eines Antikörpers, der das spezifische Protein spezifisch erkennt, durch Herstellen eines DNA-Fragments als eine auf dem DNA-Chip in Spots aufzutragende Sonde, umfassend eine Gen-codierende Region auf der genomischen DNA, Herstellen eines fluoreszenzmarkierten DNA-Fragments als ein Ziel, umfassend eine codierende Region, Hybridisierenlassen des Ziels an die Sonde, wodurch Fluoreszenzintensitätsdaten von dem Kontrolltest erhalten werden;
Eingeben der ersten und zweiten Fluoreszenzintensitätsdaten und von Informationen über die Sonden und die Ziele;
Nachweisen einer Bindungsstelle auf der genomischen DNA der Sonden, an die das spezifische Protein bindet, basierend auf den ersten und zweiten Fluoreszenzintensitätsdaten und der Information über die Sonden und die Ziele, und dann visuelles Anzeigen der Bindungsstelle auf der Genomsequenz der Sonden;
Nachweisen eines Kandidaten für ein cis-Element auf der genomischen DNA, woran das spezifische Protein bindet; und
Nachweisen der Auftrittshäufigkeit des cis-Elements in einer Gen-nicht-codierenden Region auf der genomischen DNA.

2. Verfahren zum Identifizieren einer Proteinbindungsstelle nach Anspruch 1, weiterhin umfassend ein Anzeigen einer Liste der Basensequenzen von Gen-nicht-codierenden Regionen auf der genomischen DNA, an die das spezifische Protein bindet.

3. Verfahren zum Identifizieren einer Proteinbindungsstelle nach Anspruch 1 oder 2, wobei der Anzeigeschritt der Bindungsstelle umfasst:
Durchführen einer vielfachen Ausrichtung ("multiple alignment") auf der Sequenz der Sonde im Hinblick auf die Genomsequenz des Ziels, unter Verwendung von den ersten Fluoreszenzintensitätsdaten, um den Ort der Genomsequenz des Ziels auf der Sequenz der Sonde zu identifizieren;
Vergleich der Fluoreszenzintensität der Sequenz der Sonde an dem so identifizierten Ort mit einem Schwellenwert; und
visuelles Anzeigen der nicht-codierenden Region der Sequenz der Sonde mit einer spezifischen Farbe, wenn die Fluoreszenzintensität größer als der Schwellenwert ist.

4. Verfahren zum Identifizieren einer Proteinbindungsstelle nach Anspruch 1 oder 2, wobei der Anzeigeschritt der Bindungsstelle umfasst:
Durchführen einer vielfachen Ausrichtung auf der Sequenz der Sonde im Hinblick auf die Genomsequenz des Ziels, unter Verwendung der zweiten Fluoreszenzintensitätsdaten, um den Ort der Genomsequenz des Ziels auf der Sequenz der Sonde zu identifizieren;
Vergleich der Fluoreszenzintensität der Sequenz der Sonde an dem so identifizierten Ort mit einem Schwellenwert; und
Visuelles Anzeigen der codierenden Regionen auf beiden Seiten der nicht-codierenden Region der Sequenz der Sonde mit einer spezifischen Farbe, wenn die Fluoreszenzintensität größer als der Schwellenwert ist.

5. Verfahren zum Identifizieren einer Proteinbindungsstelle nach einem der vorangehenden Ansprüche, wobei die Information über die Sonden, die in dem Dateneingabeschritt eingegeben wird, die Basensequenz der Sonden und Positionsinformation über die Sonden auf der genomischen DNA umfasst, und wobei die Information über die Ziele die Genomsequenz von jedem Chromosom der in einem relevanten Experiment verwendeten Spezies, und den Ort einer Gen-codierenden Region für jedes Chromosom umfasst.

6. Verfahren zum Identifizieren einer Proteinbindungsstelle nach einem der vorangehenden Ansprüche, wobei der Nachweisschritt der cis-Element-Häufigkeit das Herausziehen von cis-Elementen, die bestimmte Bedingungen erfüllen, unter Verwendung von MEME (Multiples EM für Motiv-Identifizierung), basierend auf einem EM-Algorithmus, umfasst, wobei die Bedingungen die maximale Anzahl an herausgezogenen cis-Elementen, ihre Länge und die Anzahl an in jeder herausgezogenen Sequenz enthaltenen Stellen umschließen.

7. Verfahren zum Identifizieren einer Proteinbindungsstelle nach einem der vorangehenden Ansprüche, weiterhin umfassend die Bestimmung von falschen Positiven auf jedem cis-Element, basierend auf der Auftrittsfrequenz von spezifischen cis-Elementen, die in dem Nachweisschritt der cis-Element-Häufigkeit erhalten werden.

8. Verfahren zum Identifizieren einer Proteinbindungsstelle nach Anspruch 7, wobei der Bestimmungsschritt von falschen Positiven umfasst:
Nachweisen von Sequenzen aus recherchierten Sequenzen, die ein oder mehrere cis-Elemente enthalten, die herausgezogen werden sollen, und Bestimmen, ob ein bestimmtes cis-Element ein falsches Positives ist, wenn die Anzahl der nachgewiesenen Sequenzen ein bestimmtes Verhältnis im Hinblick auf die recherchierten Sequenzen überschreitet;
Nachweisen von Sequenzen aus recherchierten Sequenzen mit Fluoreszenzintensitäten niedriger als eine bestimmte Fluoreszenzintensität, die ein oder mehrere cis-Elemente enthalten, die herausgezogen werden sollen, und Bestimmen eines bestimmten cis-Elements als ein falsches Positives, wenn die Anzahl der nachgewiesenen Sequenzen ein bestimmtes Verhältnis im Hinblick auf die recherchierten Sequenzen überschreitet; und
Bestimmen eines bestimmten cis-Elements als ein Positives, wenn die Anzahl an cis-Elementen, die aus einer einzelnen Recherchesequenz herausgezogen werden sollen, eine bestimmte Zahl überschreitet, wodurch bestimmt wird, dass die Sequenz durch das spezifische Protein kontrolliert wird.

9. Verfahren zum Identifizieren einer Proteinbindungsstelle nach Anspruch 7 oder 8, wobei der Schritt der Bestimmung der falschen Positiven das Anzeigen des cis-Elements umfasst, von dem bestimmt worden ist, dass es ein falsches Positives ist, mit einer Farbe, die sich von der Farbe unterscheidet, mit der das cis-Element, von dem bestimmt worden ist, dass es ein Positives ist, angezeigt wird.

10. Verfahren zum Identifizieren einer Proteinbindungsstelle nach einem der Ansprüche 7 bis 9, wobei die Schritte der Bestimmung von falschen Positiven das Erzeugen einer Tabelle von Gennamen und Auftrittshäufigkeiten von cis-Elementen umfasst, die die recherchierten Sequenzen enthalten, die stromauf von Genen enthalten sind, wobei einzelne Spalten in der Tabelle beim Sortieren in entweder aufsteigender oder absteigender Reihenfolge das Ergebnis der Bestimmung von Kontrollgenen und der Bestimmung von falschen Positiven im Hinblick auf cis-Elemente unter Verwendung von Farbschattierungen hinweisen.

## Revendications

1. Procédé d'identification d'un site de liaison de protéine dans l'ADN d'un génome, comprenant :
préparer un fragment d'ADN en tant que sonde à placer sur une puce ADN comprenant une région de gène non codant de l'ADN du génome, préparer un fragment d'ADN marqué par fluorescence en tant que cible comprenant une région de gène non codant sur l'ADN du génome, une protéine spécifique étant liée par réticulation audit fragment d'ADN, extraire le fragment d'ADN en utilisant un anticorps reconnaissant spécifiquement la protéine spécifique, supprimer la réticulation, séparer la protéine spécifique du fragment d'ADN, marquer le fragment d'ADN par un colorant fluorescent, fournissant ainsi une cible et amenant la cible à s'hybrider avec la sonde, fournissant ainsi des premières données d'intensité de fluorescence ;
préparer un fragment d'ADN en tant que sonde à placer sur la puce ADN comprenant une région de gène codant de l'ADN du génome, préparer un fragment d'ADN marqué par fluorescence en tant que cible comprenant une région de gène non codant et une partie de régions codantes des deux côtés de l'ADN, une protéine spécifique étant liée par réticulation audit fragment d'ADN, extraire le fragment d'ADN en utilisant un anticorps reconnaissant spécifiquement la protéine spécifique, supprimer la réticulation, séparer la protéine spécifique du fragment d'ADN, marquer le fragment d'ADN par un colorant fluorescent, fournissant ainsi une cible et amenant alors la cible à s'hybrider avec la sonde, fournissant ainsi des secondes données d'intensité de fluorescence ;
effectuer un test de contrôle sans utiliser d'anticorps reconnaissant spécifiquement la protéine spécifique, en préparant un fragment d'ADN en tant que sonde à placer sur la puce ADN comprenant une région de gène codant sur l'ADN du génome, en préparant un fragment d'ADN marqué par fluorescence en tant que cible comprenant une région codante, en amenant la cible à s'hybrider avec la sonde, fournissant ainsi des données d'intensité de fluorescence à partir du test de contrôle ;
introduire les première et seconde données d'intensité de fluorescence et des informations sur les sondes et les cibles ;
détecter un site de liaison dans l'ADN du génome des sondes auxquelles la protéine spécifique se lie, en fonction des première et seconde données d'intensité de fluorescence et des informations sur les sondes et les cibles, et afficher de façon visible le site de liaison de la séquence du génome des sondes ;
détecter un candidat pour un élément cis dans l'ADN du génome auquel la protéine spécifique se lie ; et
détecter la fréquence d'apparition de l'élément cis dans une région de gène non codant dans l'ADN du génome.

2. Procédé d'identification d'un site de liaison de protéine selon la revendication 1, comprenant en outre l'affichage d'une liste de séquences de base de régions de gène non codant dans l'ADN du génome auxquelles la protéine spécifique se lie.

3. Procédé d'identification d'un site de liaison de protéine selon la revendication 1 ou 2, dans lequel l'étape d'affichage du site de liaison comprend :
effectuer un alignement multiple sur la séquence de la sonde par rapport à la séquence de génome de la cible, en utilisant les premières données d'intensité de fluorescence pour identifier l'emplacement de la séquence de génome de la cible sur la séquence de la sonde ;
comparer l'intensité de fluorescence de la séquence de la sonde à l'emplacement ainsi identifié avec une valeur de seuil ; et
afficher de façon visible, si l'intensité de fluorescence est supérieure à la valeur de seuil, la région non codante de la séquence de la sonde avec une couleur spécifique.

4. Procédé d'identification d'un site de liaison de protéine selon la revendication 1 ou 2, dans lequel l'étape d'affichage du site de liaison comprend :
effectuer un alignement multiple sur la séquence de la sonde par rapport à la séquence de génome de la cible, en utilisant les secondes données d'intensité de fluorescence pour identifier l'emplacement de la séquence de génome de la cible sur la séquence de la sonde ;
comparer l'intensité de fluorescence de la séquence de la sonde à l'emplacement ainsi identifié avec une valeur de seuil ; et
afficher de façon visible, si l'intensité de fluorescence est supérieure à la valeur de seuil, les régions codantes de part et d'autre de la région non codante de la séquence de la sonde avec une couleur spécifique.

5. Procédé d'identification d'un site de liaison de protéine selon l'une quelconque des revendications précédentes, dans lequel les informations sur les sondes qui sont introduites à l'étape d'introduction de données comprennent la séquence de base des sondes et des informations de position sur les sondes de l'ADN du génome, et dans lequel les informations sur les cibles comprennent la séquence de génomes de chaque chromosome de l'espèce utilisée dans une expérience concernée et l'emplacement d'une région de gène codant pour chaque chromosome.

6. Procédé d'identification d'un site de liaison de protéine selon l'une quelconque des revendications précédentes, dans lequel l'étape de détection de fréquence d'élément cis comprend la récupération, en utilisant MEME (Multiple EM for Motif Elicitation) basé sur un algorithme EM, des éléments cis qui satisfont certaines conditions, ces conditions incluant le nombre maximal d'éléments cis récupérés, leur longueur et le nombre de sites contenus dans chaque séquence récupérée.

7. Procédé d'identification d'un site de liaison de protéine selon l'une quelconque des revendications précédentes, comprenant en outre la réalisation d'une détermination de faux-positif sur chaque élément cis en fonction de la fréquence d'apparition d'éléments cis spécifiques obtenue lors de l'étape de détection de fréquence d'éléments cis.

8. Procédé d'identification d'un site de liaison de protéine selon la revendication 7, dans lequel l'étape de détermination de faux-positif comprend :
détecter des séquences parmi des séquences recherchées qui contiennent un ou plusieurs éléments cis qui doivent être récupérés, et déterminer un élément cis particulier comme étant un faux-positif si le nombre de séquences détecté dépasse un rapport désigné pour les séquences recherchées ;
détecter des séquences parmi des séquences recherchées ayant des intensités de fluorescence inférieures à une intensité de fluorescence désignée qui contiennent un ou plusieurs éléments cis qui doivent être récupérés et déterminer un élément cis particulier comme étant un faux-positif si le nombre de séquences détecté dépasse un rapport désigné pour les séquences recherchées ; et
déterminer un élément cis particulier comme étant positif si le nombre d'éléments cis à récupérer à partir d'une seule séquence de recherche dépasse une valeur désignée, déterminant ainsi que la séquence est contrôlée par la protéine spécifique.

9. Procédé d'identification d'un site de liaison de protéine selon la revendication 7 ou 8, dans lequel l'étape de détermination de faux-positif comprend l'affichage de l'élément cis déterminé comme étant un faux-positif avec une couleur différente de la couleur d'affichage d'un élément cis déterminé comme étant positif.

10. Procédé d'identification d'un site de liaison de protéine selon l'une quelconque des revendications 7 à 9, dans lequel les étapes de détermination de faux-positif comprennent la création d'une table de noms de gènes et de fréquences d'apparition d'éléments cis qui contiennent des séquences recherchées contenues en amont des gènes, dans lequel des colonnes individuelles du tableau indiquent, lors d'un tri dans un ordre montant ou descendant, le résultat de la détermination des gènes de contrôle et la détermination de faux-positif concernant les éléments cis, en utilisant des dégradés de couleur.


### SEQUENCE LISTING

<110> Hitachi Software Engineering Co., Ltd.
<120> Method for determining protein binding sites
<130> PH-2486
<140> JP2004-262045
   <141> 2004-09-09
<160> 7
<170> PatentIn Ver. 2.1
<210> 1
   <211> 24
   <212> DNA
   <213> Artificial Sequence
<220>
   <223> Description of Artificial Sequence:Synthetic DNA
<400> 1
   tcgagtcaaa gcgagaatgc atgc 24
<210> 2
   <211> 24
   <212> DNA
   <213> Artificial Sequence
<220>
   <223> Description of Artificial Sequence:Synthetic DNA
<400> 2
   atcgaacaag cgggaagtgc tgct 24
<210> 3
   <211> 20
   <212> DNA
   <213> Artificial Sequence
<220>
   <223> Description of Artificial Sequence:Synthetic DNA
<400> 3
   cgtcgaagcg agaatgctag 20
<210> 4
   <211> 23
   <212> DNA
   <213> Artificial Sequence
<220>
   <223> Description of Artificial Sequence:Synthetic DNA
<400> 4
   tcgagtcaaa gcgtgaatgc atg 23
<210> 5
   <211> 23
   <212> DNA
   <213> Artificial Sequence
<220>
   <223> Description of Artificial Sequence:Synthetic DNA
<400> 5
   tcgagtcaaa gcgcgaatgc atg 23
<210> 6
   <211> 25
   <212> DNA
   <213> Artificial Sequence
<220>
   <223> Description of Artificial Sequence:Synthetic DNA
<400> 6
   attgcatcta tgctactgat ctatt 25
<210> 7
   <211> 21
   <212> DNA
   <213> Artificial Sequence
<220>
   <223> Description of Artificial Sequence:Synthetic DNA
<400> 7
   ctgatgcatc gtactataat t 21